**Europäisches Patentamt**

**European Patent Office**

**Office européen des brevets**

(19)

(11) Numéro de publication: **0 119 910**
**B1**

(12) **FASCICULE DE BREVET EUROPÉEN**

(45) Date de publication du fascicule du brevet:
**08.06.88**

(21) Numéro de dépôt: **84400474.7**

(22) Date de dépôt: **09.03.84**

(51) Int. Cl.⁴: **A 61 B 6/04**

(54) **Châssis d'examen basculant.**

(30) Priorité: **18.03.83 FR 8304483**

(43) Date de publication de la demande:
**26.09.84 Bulletin 84/39**

(45) Mention de la délivrance du brevet:
**08.06.88 Bulletin 88/23**

(84) Etats contractants désignés:
**BE DE IT NL**

(56) Documents cité:
**FR-A-2 039 118**
**FR-A-2 224 963**
**GB-A-2 026 206**
**US-A-3 306 605**
**US-A-3 473 024**

**H.W. FOX: "Master OP-AMP applictions
handbook", 1978, pages 309-329, Tab Books, USA**

(73) Titulaire: **THOMSON- CGR, 13, square Max-
Hymans, F-75015 Paris (FR)**

(72) Inventeur: **Wathelet, Daniel, THOMSON- CSF SCPI
173, bld Haussmann, F-75379 Paris Cedex 08 (FR)**

(74) Mandataire: **Grynwald, Albert, THOMSON- CSF
SCPI 19, avenue de Messine, F-75008 Paris (FR)**

EP 0 119 910 B1

LIBER, STOCKHOLM 1988

## Description

L'invention concerne un châssis d'examen basculant, notamment pour installation de radiologie ; elle a plus particulièrement pour objet de combiner les mouvements de basculement et de translation de la table en vue de simplifier les commandes et d'éviter les fausses manoeuvres.

En radiologie, il est souvent nécessaire de faire pivoter le châssis d'examen sur lequel se trouve le patient, jusqu'à des positions extrêmes voisines de $\pm$ 90°C par rapport à la position horizontale de la table d'examen. Cette table est assez longue de sorte qu'un simple mouvement basculant de celle-ci par rapport à son socle n'est pas suffisant pour lui assurer une possibilité de pivotement convenable. Une extrêmité de la table rencontrerait en effet assez rapidement le sol dans ces conditions. On connait par le brevet américain 3 473 024 des tables d'examen dont le mouvement de basculement est associé à un mouvement de translation. Mais cette association des mouvements n'y est liée qu'a une contrainte de faisabilité du basculement. L'invention permet de résoudre ce problème dans la mesure où elle propose d'une part de doter la table d'un double mouvement de rotation et de translation par rapport au socle, pour augmenter l'angle maximum de pivotement et, d'autre part, d'asservir les deux mouvements l'un à l'autre pour éliminer tout risque de fausse manoeuvre à l'utilisateur, lequel ne dispose plus que d'une seule commande: celle du pivotement.

Dans cet esprit, l'invention concerne donc un châssis d'examen basculant, notamment pour installation de radiologie, comprenant une table mobile par rapport à un socle fixe, un support basculant monté en rotation par rapport au-dit socle autour d'un axe horizontal et dans lequel ladite table est montée mobile en translation parallèlement à sa direction longitudinale, des premiers moyens moteurs commandés, agencés pour entraîner en rotation ledit support basculant par rapport audit socle et des seconds moyens moteurs commandés agencés pour déplacer en translation ladite table par rapport audit support basculant caractérisé en ce qu'un moyen générateur d'un signal de commande desdits seconds moyens moteurs est piloté par le mouvement desdits premiers moyens moteurs et en ce que ledit moyen générateur d'un signal de commande comporte un moyen de mesure de la rotation dudit support basculant dont une sortie de signal est appliquée à une entrée de consigne d'une chaîne d'asservissement desdits seconds moyens moteurs, par l'intermédiaire d'un circuit non linéaire dont la fonction de transfert impose la loi de variation qui lie la translation de la table à l'angle de rotation dudit support, ladite fonction de transfert est une fonction continue et comprend au moins un tronçon linéaire afin que le basculement dudit support basculant au voisinage d'une position correspondant à la position horizontale de la table soit accompagné d'une translation continue linéaire et progressive de ladite table.

L'invention sera mieux comprise et d'autres avantages de celle-ci apparaîtront mieux à la lumière de la description qui va suivre d'un châssis d'examen conforme au principe de l'invention, donnée uniquement à titre d'exemple et faite en référence aux dessins annexés dans lesquels:
- la figure 1 est une vue schématique du châssis d'examen, avec la table en position horizontale ;
- la figure 2 illustre le même châssis avec la table en position fortement inclinée ;
- la figure 3 est un schéma - bloc général du système de commande des moyens moteurs des deux mouvements du châssis ;
- la figure 4 représente une première courbe montrant la loi de variation nécessaire entre l'angle de rotation et l'amplitude de translation de la table pour obtenir une garde au sol contrôlée ainsi qu'une seconde courbe constituant une approximation possible de cette loi;
- la figure 5 est un schéma d'un circuit non linéaire.

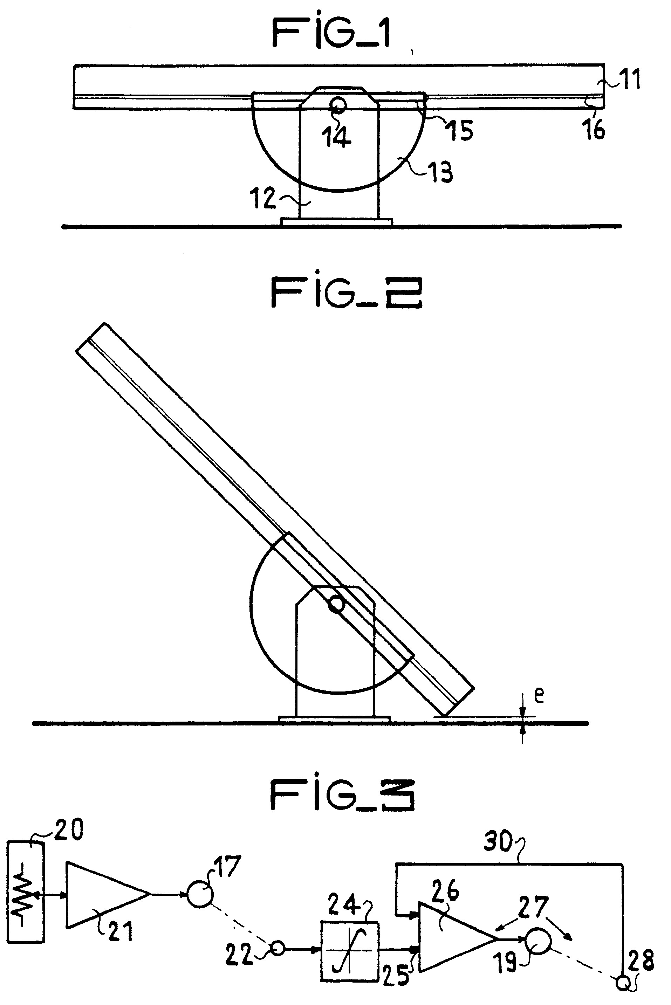

En se reportant aux figures 1 et 2, on voit que le châssis d'examen basculant selon l'invention comporte essentiellement une table 11, un socle 12, fixe, et un support basculant 13. Ce dernier est monté en rotation par rapport au socle 12 autour d'un axe 14, horizontal. Il comporte en outre deux glissières parallèles 15 situées respectivement de chaque côté de la table 11 ; elles sont engagées dans des glissières correspondantes 16 fixées le long des champs longitudinaux de ladite table. Le basculement du support 13 par rapport au socle 12 est assuré par un moteur électrique 17 (figure 3) par l'intermédiaire d'un réducteur non représenté. L'agencement de ce réducteur est classique et ne fait pas partie de l'invention ; selon un mode de réalisation actuellement préféré, il comporte un étage à courroies suivi d'un réducteur à vis et d'un étage à pignon et chaîne enroulée sur un secteur solidaire de l'axe de basculement 14. Le déplacement de la table 11 dans les glissières 15 du support basculant est aussi assuré par un moteur électrique 19 (figure 3) par l'intermédiaire d'un autre réducteur. La figure 2 illustre le double mouvement que doit effectuer la table pour pouvoir être redressée presque complètement sans toucher le sol. Le mouvement de basculement du support 13 doit s'accompagner d'une translation automatique de la table 11 par rapport à ce même support, pour toujours conserver une garde au sol minimum e. Ce double mouvement est assuré par le système de commande schématisée à la figure 3. Le moteur 17 mentionné plus haut est piloté par un circuit de commande de vitesse 21, connu, dont un organe d'affichage de consigne 20 (potentiomètre ou tout autre moyen analogue) constitue le seul moyen de règlage à la disposition de l'utilisateur. Un moyen de mesure de rotation 22 (lequel peut être un simple potentiomètre) est mécaniquement couplé à l'axe 14 ou à un point choisi du réducteur de basculement mentionné plus haut. Il élabore un signal électrique exploitable représentif du basculement du support 13 et ce signal est appliqué, via un circuit non linéaire 24, à l'entrée de consigne 25 d'une chaîne d'asservissement de position 27 de la table 11 par rapport au support basculant 13. La chaîne d'asservissement pilote bien entendu le moteur 19 et comporte un circuit de commande 26 dudit moteur, à entrée différentielle et un moyen de mesure 28 de la position de la table par rapport audit support basculant (lequel moyen de mesure peut être un potentiomètre) mécaniquement couplé au moteur 19 et élaborant un signal électrique exploitable réinjecte en tant que contre-réaction (liaison 30) à l'entrée différentielle du circuit de commande 26. La vitesse de basculement maximum est choisi suffisamment faible pour que l'asservissement de position puisse "suivre" sans retard et qu'à tout moment l'extrémité de la table ne risque pas de s'abaisser au dessous de la garde au sol $\underline{e}$ prédéterminée.

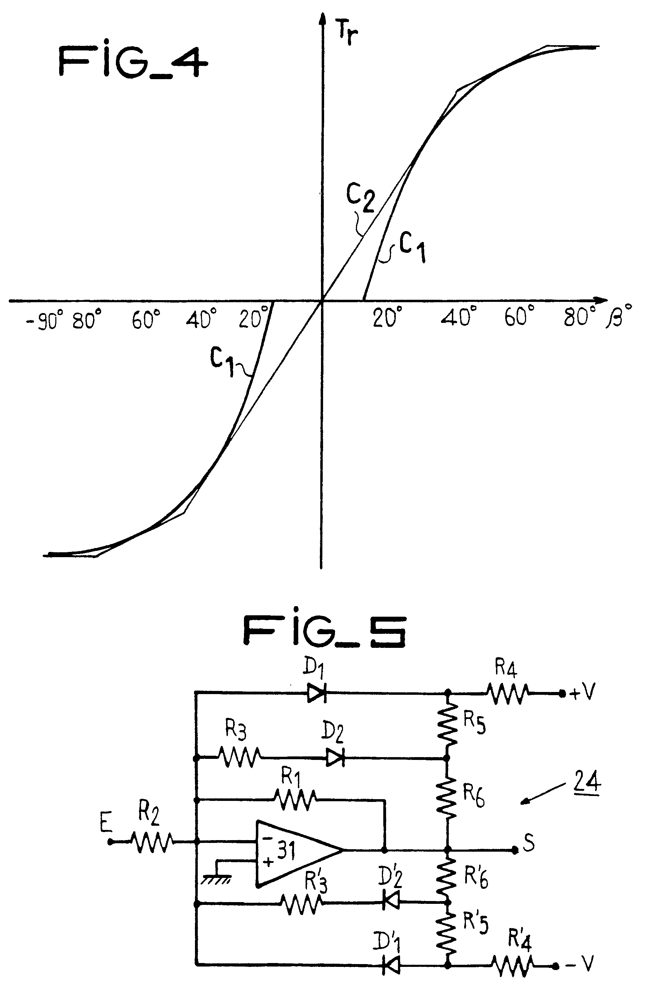

La figure 4 illustre comment déterminer la fonction de transfert du circuit non linéaire 24. La courbe discontinue $C_1$ représente la loi de variation souhaitée de l'amplitude de translation Tr de la table 11 par rapport au support basculant en fonction de l'angle $\beta$ de rotation de ce dernier. Elle est déterminée graphiquement ou trigonométriquement en fonction des dimensions du socle et de la table (hauteur de l'axe 14 par rapport au sol, longueur de la table, etc...) et de la garde au sol $\underline{e}$ prédterminée. On définit ensuite une courbe approchée $C_2$ en imposant une continuité à l'origine afin d'assurer une certaine progressivité du mouvement au voisinage de la position horizontale. La courbe $C_2$ représente la fonction de transfert du circuit non linéaire 24. Cette courbe est constituée de tronçons linéaires de longueurs différentes obtenus en créant des variations de gain dans le circuit 24 lesquelles sont déterminées par exemple au moyen de diodes. La figure 5 est un schéma possible d'un tel circuit non linéaire. Ce circuit comporte un amplificateur opérationnel différentiel 31 dont le gain est essentiellement déterminé par le rapport entre la résistance de contre-réaction $R_1$ et la résistance d'entrée $R_2$. Une chaîne de résistances en série $R_4$, $R_5$, $R_6$ est connectée entre une source de tension positive $+V$ et la sortie S du circuit non linéaire 24. Pareillement, une chaîne de résistances en série $R'_4$, $R'_5$, $R'_6$ est connectée entre une source de tension négative $-V$ et la sortie S. Une diode $D_1$ est connectée entre le point commun des résistances $R_1$ et $R_2$ et le point commun des résistance $R_4$ et $R_5$. Une diode $D'_1$ est connectée, en sens inverse par rapport à la diode $D_1$ entre le point commun des résistances $R_1$, $R_2$ et le point commun des résistance $R'_4$ et $R'_5$. Une connexion en série d'une diode $D_2$ et d'une résistance $R_3$ est branchée entre le point commun des résistances $R_1$ et $R_2$ et le point commun des résistances $R_5$, $R_6$. Une connexion

en série d'une résistance $R'_3$ et d'une diode $D'_2$ est connectée entre le point commun des résistances $R_1$ et $R_2$ et le point commun des résistances $R'_5$ et $R'_6$, la diode $D'_2$ étant branchée en sens inverse de la diode $D_2$. Avec cet agencement, la mise en conduction des diodes $D_1$ et $D_2$ (ou des diodes $D'_1$ et $D'_2$ pour les angles négatifs) provoquent les changements de pente de la courbe $C_2$. Les longueurs des tronçons à pente constante de la courbe $C_2$ sont déterminées par les diviseurs de tension $R_4$, $R_5$, $R_6$ et $R'_4$, $R'_5$, $R'_6$.

Bien entendu, l'invention n'est pas limitée au mode de réalisation qui vient d'être décrit. En particulier, l'installation peut très bien être réalisée en technologie numérique en utilisant éventuellement des moteurs pas à pas. Le circuit non linéaire serait alors par exemple une mémoire morte constituant une table de convertion. C'est dire que l'invention couvre tous les équivalents techniques des moyens mis en jeu si ceux-ci le sont dans le cadre des revendications qui suivent.

## Revendications

1. Châssis d'examen basculant, notamment pour installation de radiologie, comprenant
   - une table (11) mobile par rapport à un socle (12) fixe,
   - un support basculant (13) monté en rotation par rapport audit socle (12) autour d'un axe horizontal (14) et dans lequel ladite table (11) est montée mobile en translation parallèlement à sa direction longitudinale,
   - des premiers moyens moteurs (17) commandés, agencés pour entraîner en rotation ledit support basculant (13) par rapport audit socle (12)
   - des seconds moyens moteurs (19) commandés, agencés pour déplacer en translation ladite table (11) par rapport audit support basculant (13),
   caractérisé en ce que
   - un moyen générateur d'un signal de commande (22, 24) desdits seconds moyens moteurs (19) est piloté par le mouvement desdits premiers moyens moteurs (17) et en ce que ledit moyen générateur d'un signal de commande comporte un moyen de mesure de la rotation (22) dudit support basculant (13) dont une sortie de signal est appliquée à une entrée de consigne (25) d'une chaîne d'asservissement (27) desdits seconds moyens moteurs (19), par l'intermédiaire d'un circuit non linéaire (24) dont la fonction de transfert (C2) impose la loi de variation qui lie la translation de la table (11) à l'angle de rotation dudit support (13), ladite fonction de transfert (C2) est une fonction continue et comprend au moins un tronçon linéaire afin que le basculement dudit support basculant (13) au voisinage d'une position correspondant à la position horizontale de la table (11) soit accompagné d'une translation continue linéaire et progressive de ladite table (11).

2. Châssis d'examen selon la revendication 1, caractérisé en ce que ledit moyen de mesure de la rotation dudit support basculant est un potentiomètre (22) couplé auxdits premiers moyens moteurs (17).

3. Châssis d'examen selon la revendication 1 ou 2, caractérisé en ce que ledit circuit non linéaire (24) comporte un amplificateur opérationnel (31) muni de plusieurs branches résistives de contreréaction en parallèle (R₁-D₁-R₃, D₂) dont certaines au moins comportent des diodes.

**Patentansprüche**

1. Schwenkrahmen filr Untersuchungen, insbesondere für eine Radiologieanlage, mit
- einem Tisch (11), der bezüglich eines festen Sockels (12) beweglich ist,
- einem Schwenkträger (13), der drehbeweglich in bezug auf den Sockel (12) um eine horizontale Achse (14) gelagert ist und worin der genannte Tisch (11) parallel zu seiner Längsrichtung translationsbeweglich gelagert ist,
- ersten Antriebsmitteln (17), die so angeordnet sind, daß sie die Drehbewegung des Schwenkträgers (13) bezüglich des Sockels (12) verursachen,
- zweiten Antriebsmitteln (19), die gesteuert werden und so angeordnet sind, daß sie eine Translationsbewegung des genannten Tisches (11) bezüglich dieses Schwenkträgers (13) verursachen,
dadurch gekennzeichnet, daß
- ein Steuersignalgenerator (22, 24) zur Steuerung der zweiten Antriebsmittel (19) durch die Bewegung der genannten ersten Antriebsmittel (17) gesteuert wird und daß der Steuersignalgenerator eine Einrichtung zur Messung der Drehung (22) des Schwenkträgers (13) umfaßt, wovon ein Signalausgang an einen Sollwerteingang (25) einer Regelschleife (27) zur Nachführung der zweiten Antriebsmittel (19) über eine nichtlineare Schaltung (24) angelegt ist, deren Übertragungsfunktion (C2) das Variationsgesetz auferlegt, welches die Translationsbewegung des Tisches (11) mit dem Drehwinkel des Trägers (13) verknüpft, wobei diese Übertragungsfunktion (C2) eine stetige Funktion ist und wenigstens einen linearen Abschnitt umfaßt, damit die Verschwenkung des Schwenkträgers (13) in der Nähe einer Stellung, welche der Horizontalstellung des Tisches (11) entspricht, von einer kontinuierlichen linearen und progressiven Translationsbewegung dieses Tisches (11) begleitet ist.

2. Rahmen für Untersuchungen nach Anspruch 1, dadurch gekennzeichnet, daß die Einrichtung zur Messung der Drehung des Schwenkträgers ein Potentiometer (22) ist, welches an die ersten Antriebsmittel (17) angekoppelt ist.

3. Rahmen für Untersuchungen nach Anspruch 1 oder 2, dadurch gekennzeichnet, daß die genannte nichtlineare Schaltung (24) einen Operationsverstärker (31) umfaßt, der mit mehreren resistiven parallelen Rückkopplungszweigen (R₁-D₁-R₃, D₂) versehen ist, wovon wenigstens bestimmte Dioden enthalten.

**Claims**

1. A tiltable examination frame, more especially for radiological equipment, comprising
-a table (11) able to be moved in relation to a fixed base (12),
-a rocking support (13) pivotally mounted in relation to the said base (12) for turning about a horizontal axis (14) and which said table (11) is mounted so as to be able to be moved in translation in parallelism to its longitudinal direction,
-first controlled driving means (17) operated in order to cause rotation of the said rocking support (13) in relation to the said base (12),
-second controlled driving means (19) operated in order to displace the said table (11) in translation in relation to the said rocking support (13),
characterized in that
-a means (22 and 24) for generating a control signal for the said second driving means (19) is responsive to the movements of the said first drive means (17) and in that the said control signal generating means comprises means for measuring the rotation (22) of the said rocking support (13), of which one signal output terminal is applied to a control quantity input (25) of a control loop (27) of the said second driving means (19) through the intermediary of a non-linear circuit (24) whose transfer function (C2) establishes the variation function which links the translation of the table (11) with the angle of rotation of the said support (13), the said transfer function (C2) being a continuous function and comprising a linear extent in order to ensure that the rocking of the said rocking support (13) in the vicinity of one position corresponding to the horizontal position of the table (11) be accompanied by a continuous and progressive linear translation of the said table (11).

2. The tiltable examination frame as claimed in claim 1 characterized in that the said means for measuring the rotation of the said rocking support is a potentiometer (22) coupled with the said drive means (17).

3. The tiltable examination frame as claimed in claim 1 or claim 2 characterized in that the said non-linear circuit (24) comprises an operational amplifier (31) having a number of resistive feedback paths (R₁-D₁-R₃, D₂) in parallel of which at least some comprise diodes.

# FIG_1

11

14    15    16

13

12

# FIG_2

e

# FIG_3

20

17

21    22    24    25    26    27    19    28    30

1

FIG_4

FIG_5

3